# EUROPEAN PATENT SPECIFICATION

(11) **EP 0 786 002 B1**
(45) Date of publication and mention of the grant of the patent: **31.08.2005**
(21) Application number: 95936482.9
(22) Date of filing: 12.10.1995
(51) Int. Cl.: C12N 15/24, C07K 14/54, C12N 5/10, A61K 38/20

(54) **INTRACELLULAR ISOFORM OF THE INTERLEUKIN-1 RECEPTOR ANTAGONIST**
INTRAZELLULÄRE ISOFORM VON INTERLEUKIN-1 REZEPTOR ANTAGONIST
ISOFORME INTRACELLULAIRE DE L'ANTAGONISTE DU RECEPTEUR DE L'INTERLEUKINE-1

(30) Priority: 13.10.1994 IT MI942097
(43) Date of publication of application: 30.07.1997
(73) Proprietor: Applied Research Systems ARS Holding N.V., Curacao (AN)
(72) Inventor: COLOTTA, Francesco, I-20159 Milan (IT); MUZIO, Marta, I-20152 Milan (IT); MANTOVANI, Alberto, I-20146 Milan (IT)
(74) Representative: Lee, Nicholas John
(86) International application number: PCT/EP1995/004023
(87) International publication number: WO 1996/012022

(56) References cited:
- WO-A-91/17249
- PROCEEDINGS OF THE NATIONAL ACADEMY OF SCIENCES OF USA, vol. 88, 1991 WASHINGTON US, pages 3681-3685, S. HASKILL ET AL 'cDNA cloning of an intracellular form of the human Interleukin 1 receptor antagonist associated with epithelium' cited in the application
- JOURNAL OF IMMUNOLOGY, vol. 153, July 1994 BALTIMORE US, pages 701-711, C. BUTCHER ET AL 'Comparison of two promoters controlling expression of secreted or intracellular il-1 receptor antagonist'
- IMMUNOLOGY TODAY, vol. 12, no. 11, 1991 CAMBRIDGE GB, pages 404-410, C. A. DINARELLO ET AL 'Blocking il-1 : Interleukin 1 receptor antagonist in vivo and in vitro'
- PROCEEDINGS OF THE NATIONAL ACADEMY OF SCIENCES OF USA, vol. 85, May 1988 WASHINGTON US, pages 2929-2933, N. F. ZANDER ET AL 'cDNA cloning and complete primary structure of skeletal muscle phosphorylase kinase(alpha subunit)'
- EMBL database entry SKADECYC Accession
- PROCEEDINGS OF THE NATIONAL ACADEMY OF SCIENCES OF USA, vol. 88, June 1991 WASHINGTON US, pages 5232-5236, S.P. EISENBERG ET AL 'INterleukin 1 receptor antagonist is a member of the interleukin 1 gene family : Evolution of a cytokine control mechanism'
- JOURNAL OF EXPERIMENTAL MEDICINE, vol. 182, no. 2, 1 August 1995 pages 623-628, M. MUZIO ET AL 'Cloning and characterization of a new isoform of the interleukin 1 receptor antagonist'

## Description

### FIELD OF THE INVENTION

The present invention is in the field of biotechnology. A new interleukin-1 (IL-1) antagonist active both against IL-1a and IL-1B, a new DNA sequence encoding the IL-1 antagonist and a method for obtaining a IL-1 antagonist by recombinant DNA techniques are described. The prophylactic, therapeutic and diagnostic uses of such new IL-1 antagonist in pathologies deriving from the IL-1 production are also described.

### BACKGROUND OF THE INVENTION

There are two distinct genes encoding the interleukin-1 (IL-1) named IL-1a and IL-1B, which encode protein IL-1a (IL-1α) and IL-1B (IL-1β) respectively.

Interleukin IL-1a and IL-1B are pleiotropic cytokines, which, although their sequences show scarce homology, exert a variety of similar effects on different tissues and act on many human pathologies, in particular on the immune response of the organism and on inflammatory processes.

Both the proteins have a molecular weight of about 17.5 KDa and are synthesised as precursor molecule of larger size having a molecular weight of about 31 KDa.

IL-1s are potent inflammatory and pyrogenic cytokines that normally have beneficial effects but can also have extremely unhealthy effects for the organism.

They can, for example, participate in the pathogenesis of symptoms of autoimmune pathologies like lupus erythematosus and, in particular, they are involved as mediators which provoke damage to tissues as for example in rheumatoid arthritis.

Many of the biological effects of IL-1 are similar to those that can be observed during a septic event. Recent studies demonstrated that the endovenous administration of IL-1 in doses from 1 to 10 ng/kg gives rise to fever, sleepiness, anorexia, generalised myalgia, arthralgia and cephalea.

Since IL-1 has pleiotropic biological activities, many of which influence negatively the organism, the powerful effects of IL-1 should be under strict physiological control.

IL-1 synthesis is inhibited by anti-inflammatory cytokines, prostaglandins and glucocorticoids and the existence of multiple levels of inhibition of IL-1 points to the necessity of a strict control of this mediator.

IL-1 is the only cytokine for which an antagonist polypeptide for the receptor has been described up to now: the third known component of the IL-1 family is the antagonist for the IL-1 receptor (IL-1ra).

All three components (IL-1a, IL-1B, IL-1ra) recognise and bind to the same receptor on the cell surface (IL-1R); IL-1a and IL-1B binding to IL-1R transmit a signal, whilst IL-1ra does not.

There are two types of IL-1 receptors named IL-1RI and IL-1RII. IL-1ra is a polypeptide which binds IL-1RI, and with less affinity IL-1RII, without any agonistic activity.

IL-1ra production is induced in different cellular types, including mononuclear phagocytes, polymorphonuclear cells (PMN) and fibroblasts, by IgG, cytokines and bacterial products.

Until now two molecular forms of IL-1ra have been identified and cloned: 1) secreted IL-1ra (sIL-1ra) contains a classical leader sequence of 25 amino acids giving a mature protein of 152 amino acids; 2) intracellular IL-1ra (icIL-1ra) lacks a leader sequence thus predicting that this protein remains intracellular. Haskill et al, Proc. Natl. Acad. Sci., 1991, 88; 3681-3685 discloses the two forms of IL-1 receptor antagonist.

sIL-1ra and icIL-1ra are generated from the same gene. icIL-1ra transcripts originate from an alternative starting site and from the splicing of a first alternative exon into an internal splice acceptor site located in the first exon of sIL-1ra. The predicted proteins are thus identical except in their NH₂ ends, where the first 21 amino acids of sIL-1ra are substituted by four amino acids in icIL-1ra.

Expression of transcripts encoding sIL-1ra and icIL-1ra is differently regulated. The biological significance of icIL-1ra is still unclear.

Considering that IL-1 is involved in pathogenesis of many diseases, the need of having available medicaments useful to limit the unhealthy effects of IL-1 is evident.

### SUMMARY OF THE INVENTION

An object of the present invention is to provide an IL-1 antagonist active against both IL-1a and IL-1B and against a combination of them.

A further object of the present invention is to provide a DNA sequence encoding an IL-1 antagonist and a method for obtaining such new antagonist by the recombinant DNA technique.

Another further object of the present invention is to provide the antagonist in substantially purified form in order to be suitable for use in pharmaceutical compositions active in pathologies that require IL-1 inhibition.

Further objects and advantages of the invention will be evident in the following description.

In a first aspect, the present invention provides a substantially pure protein having antagonist activity against at least one of interleukin-1α and interleukin-β and comprising the amino acid sequences of SEQ ID NO:12. The protein may comprise the amino acid sequence of SEQ ID NO:14.

In a second aspect, the present invention provides isolated DNA encoding a protein having the amino acid sequence of SEQ ID NO:14. The DNA may have the sequence of SEQ ID NO:13.

The invention also provides a vector comprising the DNA of the second aspect and a host cell transfected with the DNA of the second aspect. The host cell may be of mammalian origin.

The invention also provides a process for obtaining an IL-1 antagonist comprising:
a. cultivating a host cell in accordance with the invention; and
b. collecting and isolating the purified protein.

The invention also provides (i) the protein of the first aspect of the invention for use in medicine, and (ii) the use of a protein of the first aspect of the invention in the preparation of a pharmaceutical compound for use in the prophylactic and/or therapeutic treatment of rheumatoid arthritis, septic shock, acute myelomonocytic leukaemia, immunological reaction of transplant against host, acquired immunodeficiency syndrome (AIDS) or ulcerative colitis.

### BRIEF DESCRIPTION OF THE FIGURES AND SEQUENCE LISTING

Figure 1 describes the DNA sequence and the sequence of the protein, for the portion not in common, of icIL-1rall (SEQ ID NO:8 and SEQ ID NO:9) compared to those of classic sIL-1ra (icIL-1ral; SEQ ID NO:6) and of sIL-1ra (SEQ ID NO:4 and SEQ ID NO:5), and it further describes the DNA sequence and the encoded protein for the portion of IL-1 ra in common (SEQ ID NO: 13 and SEQ ID NO: 14).
Figure 2 describes the RT-PCR analysis of icIL-1rall expression in different cell types.
Figure 3 describes the Western blot analysis of recombinant icIL-1rall.
Figure 4 describes the effects of icIL-1rall on IL-1 induced expression of E-selectin in endothelial cells.

SEQ ID NO: 1 reports the sequence of an oligonucleotide named IRA5 for use in RT-PCR.
SEQ ID NO:2 reports the sequence of an oligonucleotide, corresponding to nucleotides 60-79 of B-actin cDNA, for use in RT-PCR.
SEQ ID NO:3 reports the sequence of a backward oligonucleotide, complementary to nucleotides 430-449, for use in RT-PCR.
SEQ ID NO:4 reports the DNA sequence encoding sIL-1ra for the portion not in common.
SEQ ID NO:5 reports the amino acid sequence of sIL-1ra for the portion not in common.
SEQ ID NO:6 reports the DNA sequence encoding three amino acids of icIL-1ral for the portion not in common.
SEQ ID NO: 7 reports the three amino acids of icIL-1ral for the portion not in common.
SEQ ID NO:8 reports the DNA sequence encoding icIL-1rall for the portion not in common.
SEQ ID NO:9 reports the amino acid sequence of icIL-1rall for the portion not in common.
SEQ ID NO:10 reports the DNA sequence encoding IL-1ra for the portion in common. With regard to questions related to the "Patentin EPO" program, for the preparation of the sequences a G nucleotide was added in the first position of the sequence in order to permit the encode of the first amino acid Glu and further in order to avoid the formation of a stop codon in the inner side of the sequence.
SEQ ID NO: 11 reports the amino acid sequence of IL-1 ra for the portion in common.
SEQ ID NO:12 reports the sequence of 21 amino acids representing a icIL-1rall fragment not in common with the other IL-1 ras.
SEQ ID NO:13 reports the DNA sequence encoding the complete icIL-1rall.
SEQ ID NO: 14 reports the amino acid sequence of complete icIL-1rall.

### DESCRIPTION OF THE INVENTION

The new IL-1 antagonist of the invention was generated by inserting in the frame of the DNA encoding icIL-1ra a new 63 base pairs (bp) sequence between the first icIL-1ra specific exon and the internal acceptor site of the first exon of sIL-Ira.

By RT-PCR experiments the present inventors found that this new transcript is expressed in activated monocytes and fibroblasts and in polymorphonuclear cells (PMN).

Expression in COS cells revealed that this new antagonist is mostly intracellular and has a molecular weight (MW) of approximately 25 KDa in SDS-PAGE.

The new recombinant antagonist shows IL-1 inhibitory activity.

In the present application, for reason of clearness and easiness, the presently known icIL-1ra are indicated as icIL-1ra type 1 (icIL-1ral), whereas the new antagonist of the present invention is defined as icIL-1ra type II (icIL-1rall).

Examples of pathologies in which the new antagonist according to the invention can be advantageously used for prophylactic, therapeutic or diagnostic use are rheumatoid arthritis, septic shock, acute myelomonocytic leukaemia, immunological reaction of transplantation against host, acquired immunodeficiency syndrome (AIDS), ulcerative colitis and all autoimmune diseases in general.

A pharmacologically active amount of icIL-1rall can be administered to people having a high risk of developing pathologies requiring IL-1 inhibition or to people already showing pathologies like sepsis.

An example of the category above cited are patients waiting for a surgical operation.

Any route of administration compatible with the active agent can be used, but particularly preferred is the parenteral administration because it permits, in short times, systemic effects.

For this reason, the administration of a endovenous bolus just before, during or after the surgical operation is preferable. The dose of icIL-1 rall to be administered depends on the basis of the medical prescriptions according to age, weight and the individual response of the patient.

The dosage can be between 0.05 and 30 mg/Kg body weight and the preferable dose is between 0.1 and 10 mg/Kg body weight.

The pharmaceutical composition for parenteral use can be prepared in injectable form comprising the active agent and a suitable vehicle. Vehicles for parenteral administration are well known in the art and comprise, for example, water, saline solution, Ringer solution and dextrose.

The vehicle can contain smaller amounts of excipients in order to maintain the stability and isotonicity of the solution.

The preparation of the cited solutions can be carried out according to the ordinary modalities and preferably the icIL-1rall content will be comprised between 1 mg/ml and 10 mg/ml.

The present invention has been described with reference to the specific embodiments, but the content of the description comprises all modifications and substitutions which can be brought by a person skilled in the art without extending beyond the meaning and purpose of the claims.

In the following part some methods for obtaining the invention will be described, although equivalent materials and methods can be used. The following examples are therefore purely illustrative and non-limiting of the invention.

### EXAMPLE 1

### Cloning and characterisation of icIL-1raII

### MATERIALS AND METHODS

### Reagents

The following commercially available reagents were used for culture and separation of cells: pyrogenfree saline and distilled water for clinical use; RPMI 1640 medium; DMEM medium; M199 medium; L-glutamine; Percoll; Ficoll-Hipaque; aseptically collected fetal calf serum; endothelial cells growth supplement (ECGS), prepared from bovine brain; Heparin.

All reagents contained less than 0.125 EU/ml of endotoxin as checked by the Limulus amebocyte lysate assay.

### Cells

Human circulating PMN and monocytes were separated from the peripheral blood of healthy donors by centrifugation on a discontinuous (46% for monocytes and 62% for PMN) gradient of isoosmotic (285 mOsm) Percoll, as described in Colotta F., Peri G., Villa SA., Mantovani A., Rapid killing of actinomycin D treated tumour cells by human mononuclear cells. J. Immunol. 132:936, 1984. Cells were recovered at the interface, washed twice in saline and resuspended in the medium.

PMN and monocytes recovery was higher than 90% and purity higher than 98%, as assessed by morphological examination of stained cytocentrifuged cells. The cell culture medium routinely used for PMN and monocytes was RPMI 1640 with 2 mM L-glutamine and 10% FCS.

Human endothelial cells (EC) were obtained from umbilical veins and cultured, as described in detail in the literature (Allavena P., Paganin C., Martin-Padura L, Peri G., Gaboli M., Dejana E., Marchisio P.C., Mantovani A., Molecules and structures involved in the adhesion of natural killer cells to vascular endothelium, J. Exp. Med., 173:439, 1991).

Confluent cells at 2nd-5th passage maintained in M199 medium with 10% FCS supplemented with ECGS (50 µg/ml) and Heparin (100 µg/ml) were routinely used.

COS cells were cultivated in DMEM medium with 10% FCS and 8387 fibroblast cells in RPMI 1640 medium with 10% FCS.

After the appropriate treatment, cells were examined for IL-1ra mRNA or IL-1ra protein as described below.

### RT-PCR

Total RNA was extracted by the guanidinium isothiocianate method with minor modifications.

RT-PCR was performed as described in Colotta F., Polentarutti N., Sironi M., Mantovani A., J. Biol. Chem., 267:18278, 1992.

Briefly, 1 µg total RNA was reverse transcribed in reverse transcriptase buffer (5 mM MgCl₂, 50 mM KCI, 10 mM Tris-HCl; pH 8.3) with 2.5 mM random hexamers, 1 mM each deoxynucleotide triphosphate, 1 unit/ml RNase inhibitor, and 2.5 units/ml moloney murine leukaemia virus transcriptase (Perkin Elmer Cetus, Norwalk, CT).

Samples were incubated for 10 min at 25°C and then at 42°C for 45 min. Then, cDNA reaction was added with a specific pair of primers designed to amplify cDNAs encoding icIL-1ral or icIL-1raII and, as an internal control, human B-actin.

Amplification was carried out in 2 mM MgCl₂, 50 mM KCI, 0.2 M each deoxynucleotide triphosphate, 2.5 units/100 ml Taq polymerase (Perkin Elmer Cetus) and 4 mg/ml of each specific primer (see below). Amplification (30 cycles) was carried out in an automated thermal cycler (Perkin Elmer Cetus) at 95°C, at 55°C and at 72°C for 1.5 min each.

Amplified products were run through a 1% ethidium bromide-stained agarose gel along with molecular weight standards (Boehringer Mannheim, Mannheim, Germany).

Oligonucleotides were synthesised by the phosphoramidite method. The sequences of oligonucleotides used to selectively amplify icIL-1ra were identical to those described in Haskill S. et al., Natl. Acad., USA, 88:3681, 1991.

In particular, the authors used oligonucleotides GM397 (indicated here as IRA 1) and GM368 (IRA 4).

For icIL-1raII amplification the authors used IRA 4 and IRA 5 (SEQ ID NO:1), which specifically recognises the extra exon described here included in the icIL-1raII sequence.

For B-actin amplification the forward oligonucleotide is reported in SEQ ID NO:2, corresponding to nucleotides 60-79 of B-actin cDNA.
The backward oligonucleotide is reported in SEQ ID NO:3, complementary to nucleotides 430-449. Amplification products were subcloned (TA Cloning System, Invitrogen, San Diego, CA) and sequenced by the dideoxy chain termination method.

### Expression of icIL-Ira products in COS cells

The cDNAs containing 32 bp of the 5'-untranslated region, the complete open reading frame and 6 bp (including the stop codon) of the 3'-untranslated region of both the icIL-1ral and icIL-1rall were obtained by RT-PCR with oligonucleotides IRA 4 and IRA 5 as detailed above and then ligated back into the pSF5 expression vector. Fidelity of reverse transcription and amplification was verified by sequencing.

The plasmids containing the cDNA in the correct orientation were purified on a CsCl gradient and then transfected into COS cells by the calcium precipitate method as described in Sambrook J. et al., Cold Spring Harbor Laboratory Press, 1989.

After two days, culture supernatants and sonicated cell lysates were examined by ELISA or immunoblotting as detailed below. An empty plasmid (not transfected) was used as a control.

### Identification of immunoreactive IL-1ra

A commercial ELISA test (Amersham, Buckinghamshire, UK) that identifies both sIL-1ra and icIL-1ra was used. For the Western blot analysis polyclonal antisera of two rabbits and of one goat were used.

COS cells lysates samples and supernatants were run on 12.5% SDS-PAGE electrophoresis and then blotted onto a nitro-cellulose filter (Stratagene, La Jolla, CA, USA).

Incubation with primary and secondary antibodies was carried out according to standard protocols. The primary antibody was an anti-IL-1ra rabbit polyclonal antibody.

The secondary antibody was a goat anti-rabbit immunoglobulin fraction linked to horseradish peroxidase (Amersham). Immunoreactive protein fraction bands were revealed by a chemiluminescence-based procedure (ECL Detection, Amersham) according to manufacturer's instructions.

### II-1-induced expression of E-selectin on EC

Confluent EC cultivated in 96 well plates (Falcon) were incubated for 30 minutes with an amount of transfected COS cells lysate (see above) corresponding to 25 to 100 ng of recombinant IL-1ra (either icIL-1raI or icIL-1raII) as assessed by a specific ELISA assay (Amersham).

As a control, an equal amount of COS lysate obtained from mock transfected cells was used in parallel. Next, EC were exposed for 6 hours to 0.1-1 ng/ml human recombinant IL-1B. The detection of E-selectin expression was made with an ELISA assay on adherent EC with the anti-E-selectin monoclonal antibody BB1G-E2 as primary antibody and a rabbit anti-mouse Ig antiserum conjugated with horseradish peroxidase as a secondary antibody. O.D. of the samples was determined by detecting the plates with a spectrophotometer (Flow) at 405 wavelength.

### RESULTS

### Identification of icIL-1raII

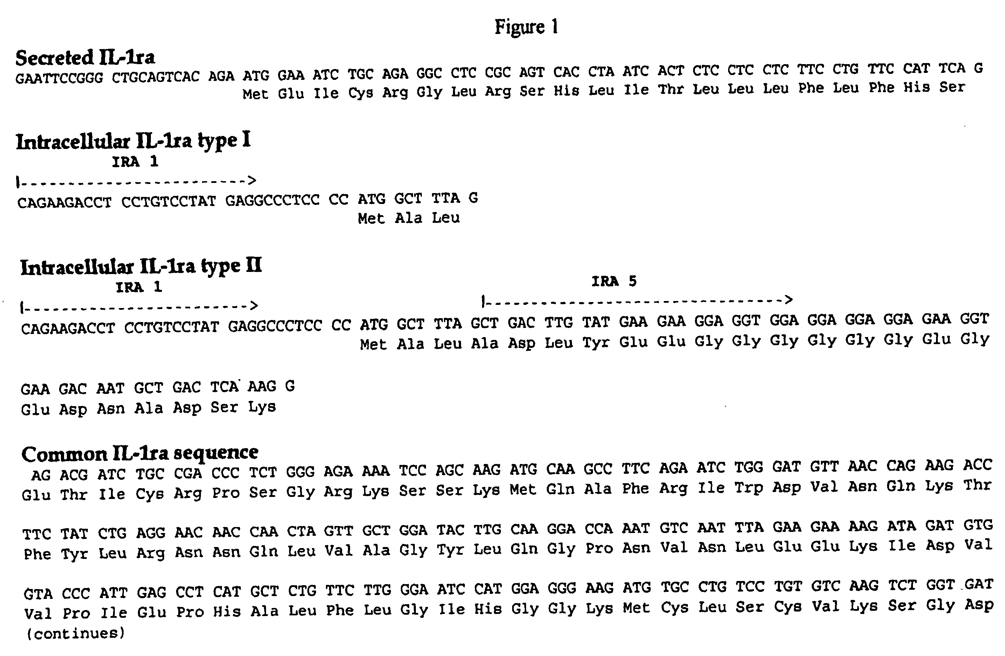

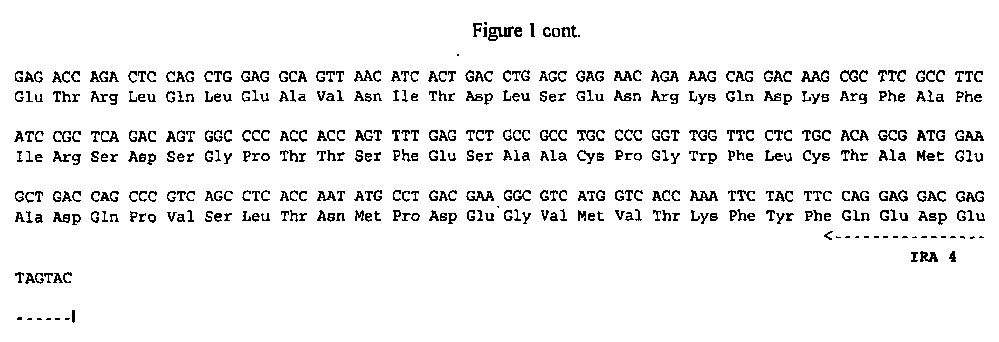

Specific oligonucleotide primers were designed (indicated as IRA 1 and IRA 4 in Fig.1) in order to obtain the whole coding sequence of icIL-1ra (Fig.1) by RT-PCR. Amplified products from human PMN were subcloned and sequenced.

In addition to the previously known sequence of icIL-1ra, the inventors isolated a number of clones whose sequences were identical to the published icIL-1ra coding sequence, with the notable exception of an extra sequence of 63 bp between nucleotides 132 and 133 of the icIL-1ra sequence. Given the described exon-intron boundaries of icIL-1ra, the extra sequence is inserted between the first leader-less exon of icIL-1ra and the internal acceptor site of the first exon of sIL-1ra (Fig.1).

The predicted amino acid sequence is shown in Fig.1. The new protein (thereafter referred to as icIL-1ra type II) has the first three amino acids at the NH₂ terminus in common with the classical icIL-1ra (icIL-1ra type I), followed by a new sequence of 21 amino acids. The rest of the two proteins is identical.

Curiously, the junction with the internal acceptor site of the first exon of sIL-1ra always generated, both for sIL-1ra and icIL-1raI and for icIL-1raII, the same amino acid residue, i.e. glutamic acid (Fig.1).

The most striking characteristic of the inserted amino acid extra sequence is the presence of seven glycine residues, six of which are consecutive. Glycine residues are flanked on both sides by glutamic acid residues. icIL-1rall consists of 180 amino acids.

The overall hydrophilic pattern of icIL-1raII is similar to that of icIL-1raI, still lacking an hydrophobic leader peptide at the NH₂ terminus.

### Expression of icIL-raII

To identify icIL-1raII transcripts, RT-PCR analysis was performed with a pair of specifically designed oligonucleotides (IRA 5 and IRA 4, Fig.1), with an expected amplified product of 33 bp.

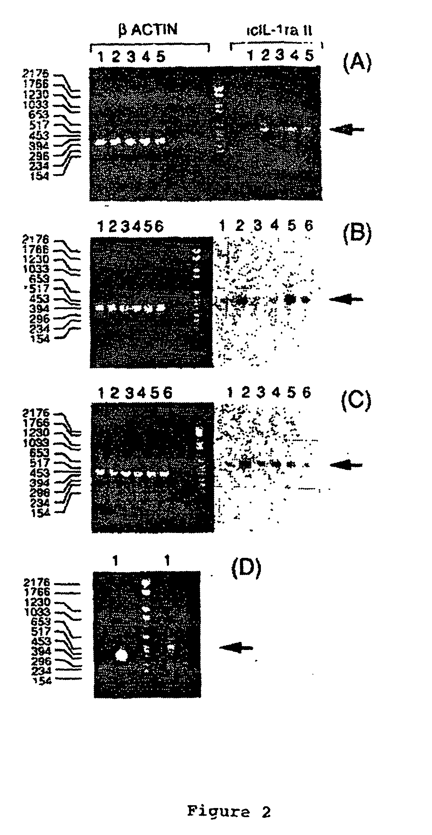

As shown in Fig.2, transcripts encoding icIL-1raII were detectable in PMA-, IL-1- and TNF-activated fibroblasts. A faint but detectable band was evident in LPS-treated monocytes.

Also PMN, either untreated or activated (Fig.2) showed a very faint band of the expected size.

The specificity of amplified products indicated in Fig.2 was confirmed by subcloning and sequencing.

### Expression of recombinant icIL-1raII

COS cells were transfected with the DNA sequence encoding icIL-1raII and, by way of comparison, with that encoding icIL-1raL Next, cell lysates and supernatants were examined by Western blot.

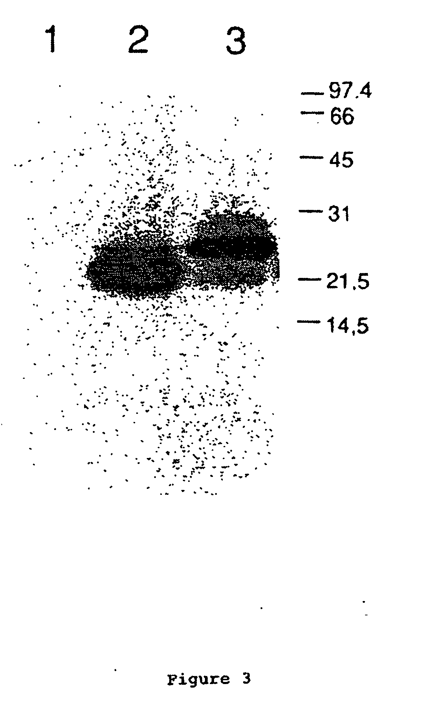

The polyclonal antisera used in these experiments recognised equally well icIL-1raII and icIL-1raI (Fig.3). Most, if not all, of icIL-1raII and icIL-1raI were found in cell lysates.

Recombinant icIL-1raI migrated as a predominant band of 22 KDa, whereas icIL-1raII showed a mass of approximately 25 KDa.

### Inhibition of IL-1B activity by recombinant icIL-1raII

Recombinant icIL-1raII was examined for IL-1 inhibiting activity. To this aim the authors chose the IL-1-induced expression of E-selectin on endothelial cells, because this assay is sensitive (detectable induction at 100 pg/ml IL-1, or less) and rapid (6 hours incubation with IL-1).

Lysates of mock transfected COS cells did not significantly reduce the IL-1 activity.

icIL-1raII had no agonistic activity.

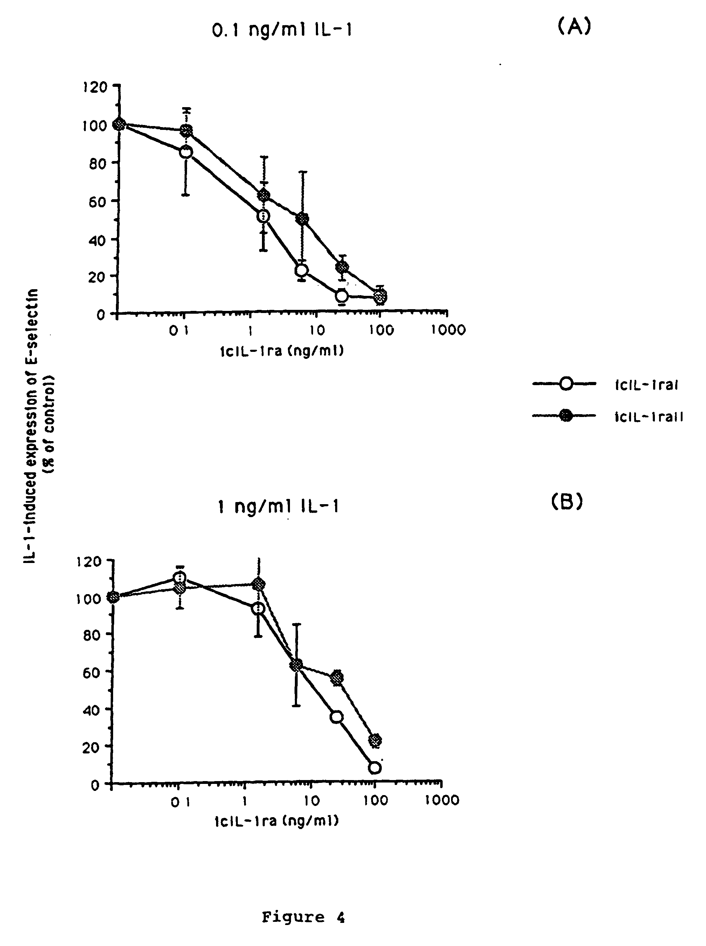

As shown in Fig.4, recombinant icIL-1raII inhibited in a dose-dependent fashion IL-1 activity.

These data provide evidence that icIL-1raII is indeed an inhibitor of IL-1.

### DISCUSSION

The inventors describe a new molecular form of icIL-1ra. The new molecule is generated by insertion of 63 bp between the first leader-less exon of icIL-1ra and the internal acceptor site of the first exon of sIL-1ra.

Since the resulting protein is partially identical to classical icIL-1ra, with the exception of an extra sequence of 21 amino acids located in the NH₂ terminus of the molucule, the inventors suggest to term this new form as IL-1ra type II, referring to the classical icIL-1ra sequence as icIL-1ra type I.

RT-PCR experiments demonstrated that icIL-1raII transcripts are inducible in monocytes and fibroblasts. Recombinant icIL-1raII expressed in COS cells had an apparent MW of approximately 25 KDa and an inhibitor activity of IL-1 comparable to that exerted by icIL-1raI expressed under the same experimental conditions.

Transcripts coding for icIL-1ra and sIL-1ra are generated from the same gene by means of usage of differential splicing. icIL-1ra is generated by an alternative start of transcription of an exon inserted into an internal acceptor site of the first exon containing the leader sequence of sIL-1ra.

The results obtained by the inventors suggest a new organisation of IL-1ra gene, in which an extra exon is located between the first exon of, respectively, classical icIL-1ra and sIL-1ra. Use of this new exon generates a polypeptide molecule which, still lacking a signal peptide, differs from icIL-1raI at its N terminus by the insertion of 21 amino acids, whilst retaining inhibitory capacity against IL-1.

Use of alternative splicing to generate different IL-1ra molecules appears to be highly regulated. icIL-1raII transcripts were induced by IL-1, TNF and phorbol esters in fibroblasts and by LPS in monocytes. In fibroblasts, phorbol esters were found to selectively induce icIL-1ra transcripts, whereas IL-1 and TNF induced both sIL-1ra and icIL-1ra mRNAs. In monocytes, IL-13, which augmented both transcripts of sIL-1ra and icIL-1raI, failed to induce icIL-1raII.

Finally, PMN, in which sIL-1ra and icIL-1ra are costitutively expressed and inducible, expressed very few transcripts, as pointed out by RT-PCR. Overall, these data indicate that the mechanisms inducing the differential splicing generating the three forms of IL-1ra are differentially regulated in response to external signals.

The amino acid sequence of the extra sequence described here is surprising in that it contains seven residues of glycine, six of which are consecutive.

Glycine-rich sequences are present in molecules with different biological activities, including the atrial natriuretic clearance receptor, the HOX11 home box gene, the intermediate filaments keratins and nuclear proteins involved in centromere binding or RNA splicing.

Apart from glycine residues, however, no obvious homology was evident between these proteins and icIL-1rall in the amino acid sequence flanking glycine-rich regions.

IL-1 system shows an extraordinary level of complexity, consisting of two agonists, two receptors, one of which is an inhibitor of IL-1, and a receptor antagonist, for which at least three different molecular forms could exist taking into account the results obtained.

Although the biological significance of the intracellular forms of IL-1ra remains to be clearly established, the data here reported indicate that by alternative splicing two different forms of icIL-1ra can be generated in response to selected external stimuli, with different N termini.

The existence of multiple and complex levels of control of IL-1 points to the absolute requirement for a tight physiological control of the inflammatory potential of this cytokine.

### DESCRIPTION OF FIGURES

### Figure 1

### DNA sequence and predicted protein sequence of icIL-1raII compared to classical icIL-1ra(icIL-1raI) and sIL-1ra.

The upper part of Figure 1 shows DNA and protein sequences specifically represented in sIL-1ra, icIL-1ral and icIL-1raII. The lower part of Figure 1 shows the sequence in common among the three forms of IL-1ra.

The entire sequences for each molecule are thus generated by the junction of each specific portion with the common sequence. For clarity, the DNA sequence of icIL-1ra starts from nucleotide 91 of the published 5' untranslated sequence, and only 6 bp of the 3' untranslated sequence are reported.

The common IL-1 ra sequence starts with the internal acceptor site located in the first exon of sIL-1ra, corresponding to nucleotide 133 of the complete icIL-1ral sequence and to nucleotide 88 of the complete sIL-1ra sequence.

Arrows indicate forward (IRA 1 and IRA 5) and backward (IRA 4) oligonucleotides used for RT-PCR analysis, as described in the text. The oligonucleotide IRA 5 recognises only icIL-1raII DNA.

### Figure 2

### RT-PCR analysis of icIL-1raII expression in different cell types

RNAs from 8387 fibroblasts (panel A), monocytes (B) and PMN (C) were reverse-transcribed. Each DNA synthesis reaction was then divided in two samples, one of which amplified with oligonucleotides IRA 5 (forward) and IRA 4 (backward) for detection of icIL-1raII transcripts, and the other amplified with B-actin specific oligonucleotides (see Material and Methods Section).

Amplified products were then examined through an ethidium bromide-stained agarose gel. Amplified products corresponding to B-actin are reported on the left side of the standard and the amplified products corresponding to icIL-1raII (on the right) are indicated by a arrow. The specificity of these bands was confirmed by subcloning and sequencing.

### Figure 3

### Western blot analysis of recombinant icIL-1raII

Cell lysates from COS cells transfected with DNAs encoding icIL-1raI (2) or icIL-1raII (3) or with an empty vector which does not contain such DNA (1) were examined by immunoblotting with an anti-IL-1ra rabbit polyclonal antibody. Molecular weight standards are indicated.

### Figure 4

### Effects of icIL-1raII on IL-1-induced expression of E-selectin on endothelial cells

Endothelial cells were treated with 0.1 or 1 ng/ml of human IL-1B, with or without 25-100 ng/ml of icIL-1rall or equivalent amounts of COS cell lysates obtained from cells which were mock transfected by means of an empty vector, as explained in detail in the Material and Method section.

After 6 hours of incubation, the endothelial cells were examined for E-selectin expression by an ELISA test performed on adherent cells.

The data reported are percentages of IL-1-induced E-selectin expression for the control.

### SEQUENCE LISTING

(1) GENERAL INFORMATION:
   (i) APPLICANT:
      (A) NAME: APPLIED RESEARCH SYSTEMS ARS HOLDING N.V.
      (B) STREET: 14 JOHN B. GORSIRAWEG
      (C) CITY: CURACAO
      (E) COUNTRY: NETHERLANDS ANTILLES
      (F) POSTAL CODE (ZIP): NONE
      (G) TELEPHONE: 599-9639300
      (H) TELEFAX: 599-9614129
   (ii) TITLE OF INVENTION: INTERLEUKIN-1 ANTAGONIST
   (iii) NUMBER OF SEQUENCES: 14
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: misc_feature
      (B) LOCATION:1..25
      (D)OTHER INFORMATION:/note= "RT-PCR oligonucleotide named IRA5"
   (xi) SEQUENCE DESCRIPTION: SEQ ID NO: 1:
   CTGACTTGTA TGAAGAAGGA GGTGG 25
(2) INFORMATION FOR SEQ ID NO: 2:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: misc_feature
      (B) LOCATION:1..20
      (D) OTHER INFORMATION:/note= "RT-PCR oligonucleotide corresponding to 60-79 of B-actin"
   (xi) SEQUENCE DESCRIPTION: SEQ ID NO: 2:
   GCGCTCGTCG TCGACAACGG 20
(2) INFORMATION FOR SEQ ID NO: 3:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: misc_feature
      (B) LOCATION:1..21
      (D) OTHER INFORMATION:/note= "RT-PCR backward oligonucleotide complementary to 430-449"
   (xi) SEQUENCE DESCRIPTION: SEQ ID NO: 3:
   GATAGACAAC GTACATGGCT G                                              21
(2) INFORMATION FOR SEQ ID NO: 4:
   (i) SEQUENCE CHARACTERISTICS:
      (A) LENGTH: 87 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION:24..86
   (ix) FEATURE:
      (A) NAME/KEY: misc_feature
      (B) LOCATION:1..87
      (D) OTHER INFORMATION:/note= "Sequence of sIL-1ra not in common"
   (xi) SEQUENCE DESCRIPTION: SEQ ID NO: 4:
   GAATTCCGGG CTGCAGTCAC AGA ATG GAA ATC TGC AGA GGC CTC CGC AGT      50
                             Met Glu Ile Cys Arg Gly Leu Arg Ser
                               1               5
   CAC CTA ATC ACT CTC CTC CTC TTC CTG TTC CAT TCA G                  87
   His Leu Ile Thr Leu Leu Leu Phe Leu Phe His Ser
   10                  15                  20
(2) INFORMATION FOR SEQ ID NO: 5:
   (i) SEQUENCE CHARACTERISTICS:
      (A) LENGTH: 21 amino acids
      (B) TYPE: amino acid
      (D) TOPOLOGY: linear
   (ii) MOLECULE TYPE: protein
   (xi) SEQUENCE DESCRIPTION: SEQ ID NO: 5:
   Met Glu Ile Cys Arg Gly Leu Arg Ser His Leu Ile Thr Leu Leu Leu
    1               5                  10                  15
   Phe Leu Phe His Ser
               20
(2) INFORMATION FOR SEQ ID NO: 6:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION:33..41
   (ix) FEATURE:
      (A) NAME/KEY: misc_feature
      (B) LOCATION:1..42
      (D) OTHER INFORMATION:/note= "Sequence of intracellular IL-1ra type I not in common"
   (xi) SEQUENCE DESCRIPTION: SEQ ID NO: 6:
   CAGAAGACCT CCTGTCCTAT GAGGCCCTCC CC ATG GCT TTA G             42
                                       Met Ala Leu
                                        1
(2) INFORMATION FOR SEQ ID NO: 7:
   (i) SEQUENCE CHARACTERISTICS:
      (A) LENGTH: 3 amino acids
      (B) TYPE: amino acid
      (D) TOPOLOGY: linear
   (ii) MOLECULE TYPE: protein
   (xi) SEQUENCE DESCRIPTION: SEQ ID NO: 7:
   Met Ala Leu
    1
(2) INFORMATION FOR SEQ ID NO: 8:
   (i) SEQUENCE CHARACTERISTICS:
      (A) LENGTH: 105 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION:33..104
   (ix) FEATURE:
      (A) NAME/KEY: misc_feature
      (B) LOCATION:1..105
      (D) OTHER INFORMATION:/note= "Sequence of intracellular IL-1ra type II not in common"
   (xi) SEQUENCE DESCRIPTION: SEQ ID NO: 8:
   CAGAAGACCT CCTGTCCTAT GAGGCCCTCC CC ATG GCT TTA GCT GAC TTG TAT     53
                                       Met Ala Leu Ala Asp Leu Tyr
                                        1               5
   GAA GAA GGA GGT GGA GGA GGA GGA GAA GGT GAA GAC AAT GCT GAC TCA    101
   Glu Glu Gly Gly Gly Gly Gly Gly Glu Gly Glu Asp Asn Ala Asp Ser
           10                  15                  20
   AAG G                                                              105
   Lys
(2) INFORMATION FOR SEQ ID NO: 9:
   (i) SEQUENCE CHARACTERISTICS:
      (A) LENGTH: 24 amino acids
      (B) TYPE: amino acid
      (D) TOPOLOGY: linear
   (ii) MOLECULE TYPE: protein
   (xi) SEQUENCE DESCRIPTION: SEQ ID NO: 9:
   Met Ala Leu Ala Asp Leu Tyr Glu Glu Gly Gly Gly Gly Gly Gly Glu
    1               5                  10                  15
   Gly Glu Asp Asn Ala Asp Ser Lys
               20
(2) INFORMATION FOR SEQ ID NO: 10:
   (i) SEQUENCE CHARACTERISTICS:
      (A) LENGTH: 474 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION:1..468
   (ix) FEATURE:
      (A) NAME/KEY: misc_feature
      (B) LOCATION:1..474
      (D) OTHER INFORMATION:/note= "Common IL-1ra seg.; a G was added in the first position for software reason, so as the first codon codes for Glu and so as the creation of a stop codon in the inner region of the seq. is avoided"
   (xi) SEQUENCE DESCRIPTION: SEQ ID NO: 10:
   GAG ACG ATC TGC CGA CCC TCT GGG AGA AAA TCC AGC AAG ATG CAA GCC     48
   Glu Thr Ile Cys Arg Pro Ser Gly Arg Lys Ser Ser Lys Met Gln Ala
     1               5                  10                  15
   TTC AGA ATC TGG GAT GTT AAC CAG AAG ACC TTC TAT CTG AGG AAC AAC     96
   Phe Arg Ile Trp Asp Val Asn Gln Lys Thr Phe Tyr Leu Arg Asn Asn
                20                  25                  30
   CAA CTA GTT GCT GGA TAC TTG CAA GGA CCA AAT GTC AAT TTA GAA GAA    144
   Gln Leu Val Ala Gly Tyr Leu Gln Gly Pro Asn Val Asn Leu Glu Glu
            35                  40                  45
   AAG ATA GAT GTG GTA CCC ATT GAG CCT CAT GCT CTG TTC TTG GGA ATC    192
   Lys Ile Asp Val Val Pro Ile Glu Pro His Ala Leu Phe Leu Gly Ile
        50                  55                  60
   CAT GGA GGG AAG ATG TGC CTG TCC TGT GTC AAG TCT GGT GAT GAG ACC    240
   His Gly Gly Lys Met Cys Leu Ser Cys Val Lys Ser Gly Asp Glu Thr
    65                  70                  75                  80
   AGA CTC CAG CTG GAG GCA GTT AAC ATC ACT GAC CTG AGC GAG AAC AGA    288
   Arg Leu Gln Leu Glu Ala Val Asn Ile Thr Asp Leu Ser Glu Asn Arg
                       85                       90                       95
   AAG CAG GAC AAG CGC TTC GCC TTC ATC CGC TCA GAC AGT GGC CCC ACC    336
   Lys Gln Asp Lys Arg Phe Ala Phe Ile Arg Ser Asp Ser Gly Pro Thr
               100                      105                      110
   ACC AGT TTT GAG TCT GCC GCC TGC CCC GGT TGG TTC CTC TGC ACA GCG    384
   Thr Ser Phe Glu Ser Ala Ala Cys Pro Gly Trp Phe Leu Cys Thr Ala
           115                      120                      125
   ATG GAA GCT GAC CAG CCC GTC AGC CTC ACC AAT ATG CCT GAC GAA GGC    432
   Met Glu Ala Asp Gln Pro Val Ser Leu Thr Asn Met Pro Asp Glu Gly
       130                      135                      140
   GTC ATG GTC ACC AAA TTC TAC TTC CAG GAG GAC GAG TAGTAC          474
   Val Met Val Thr Lys Phe Tyr Phe Gln Glu Asp Glu
   145
(2) INFORMATION FOR SEQ ID NO: 11:
   (i) SEQUENCE CHARACTERISTICS:
      (A) LENGTH: 156 amino acids
      (B) TYPE: amino acid
      (D) TOPOLOGY: linear
   (ii) MOLECULE TYPE: protein
   (xi) SEQUENCE DESCRIPTION: SEQ ID NO: 11:
   Glu Thr Ile Cys Arg Pro Ser Gly Arg Lys Ser Ser Lys Met Gln Ala
    1               5                  10                  15
   Phe Arg Ile Trp Asp Val Asn Gln Lys Thr Phe Tyr Leu Arg Asn Asn
               20                  25                  30
   Gln Leu Val Ala Gly Tyr Leu Gln Gly Pro Asn Val Asn Leu Glu Glu
           35                  40                  45
   Lys Ile Asp Val Val Pro Ile Glu Pro His Ala Leu Phe Leu Gly Ile
       50                  55                  60
   His Gly Gly Lys Met Cys Leu Ser Cys Val Lys Ser Gly Asp Glu Thr
   65                  70                  75                  80
   Arg Leu Gln Leu Glu Ala Val Asn Ile Thr Asp Leu Ser Glu Asn Arg
                   85                  90                  95
   Lys Gln Asp Lys Arg Phe Ala Phe Ile Arg Ser Asp Ser Gly Pro Thr
               100                 105                 110
   Thr Ser Phe Glu Ser Ala Ala Cys Pro Gly Trp Phe Leu Cys Thr Ala
   115 120 125
   Met Glu Ala Asp Gln Pro Val Ser Leu Thr Asn Met Pro Asp Glu Gly
   130 135 140
   Val Met Val Thr Lys Phe Tyr Phe Gln Glu Asp Glu
   145 150 155
(2) INFORMATION FOR SEQ ID NO: 12:
   (i) SEQUENCE CHARACTERISTICS:
      (A) LENGTH: 21 amino acids
      (B) TYPE: amino acid
      (C) STRANDEDNESS:
      (D) TOPOLOGY: linear
   (ii) MOLECULE TYPE: peptide
   (iii) HYPOTHETICAL: NO
   (iv) ANTI-SENSE: NO
   (v) FRAGMENT TYPE: N-terminal
   (ix) FEATURE:
      (A) NAME/KEY: Peptide
      (B) LOCATION:1..21
      (D) OTHER INFORMATION:/note= "A portion of the intracellular IL-1ra type II not in common"
   (xi) SEQUENCE DESCRIPTION: SEQ ID NO: 12:
   Ala Asp Leu Tyr Glu Glu Gly Gly Gly Gly Gly Gly Gly Gly Glu Asp
   1 5 10 15
   Asn Ala Asp Ser Lys
   20
(2) INFORMATION FOR SEQ ID NO: 13:
   (i) SEQUENCE CHARACTERISTICS:
      (A) LENGTH: 579 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION:34..573
   (ix) FEATURE:
      (A) NAME/KEY: misc_feature
      (B) LOCATION:1..579
      (D) OTHER INFORMATION:/note= "Intracellular IL-1ra type II"
   (xi) SEQUENCE DESCRIPTION: SEQ ID NO: 13:
   CAGAAGGACC TCCTGTCCTA TGAGGCCCTC CCC ATG GCT TTA GCT GAC TTG TAT    54
                                        Met Ala Leu Ala Asp Leu Tyr
                                          1               5
   GAA GAA GGA GGT GGA GGA GGA GGA GAA GGT GAA GAC AAT GCT GAC TCA    102
   Glu Glu Gly Gly Gly Gly Gly Gly Glu Gly Glu Asp Asn Ala Asp Ser
            10                  15                  20
   AAG GAG ACG ATC TGC CGA CCC TCT GGG AGA AAA TCC AGC AAG ATG CAA    150
   Lys Glu Thr Ile Cys Arg Pro Ser Gly Arg Lys Ser Ser Lys Met Gln
        25                  30                  35
   GCC TTC AGA ATC TGG GAT GTT AAC CAG AAG ACC TTC TAT CTG AGG AAC    198
   Ala Phe Arg Ile Trp Asp Val Asn Gln Lys Thr Phe Tyr Leu Arg Asn
    40                  45                  50                  55
   AAC CAA CTA GTT GCT GGA TAC TTG CAA GGA CCA AAT GTC AAT TTA GAA    246
   Asn Gln Leu Val Ala Gly Tyr Leu Gln Gly Pro Asn Val Asn Leu Glu
                    60                  65                  70
   GAA AAG ATA GAT GTG GTA CCC ATT GAG CCT CAT GCT CTG TTC TTG GGA    294
   Glu Lys Ile Asp Val Val Pro Ile Glu Pro His Ala Leu Phe Leu Gly
                75                  80                  85
   ATC CAT GGA GGG AAG ATG TGC CTG TCC TGT GTC AAG TCT GGT GAT GAG    342
   Ile His Gly Gly Lys Met Cys Leu Ser Cys Val Lys Ser Gly Asp Glu
            90                  95                 100
   ACC AGA CTC CAG CTG GAG GCA GTT AAC ATC ACT GAC CTG AGC GAG AAC    390
   Thr Arg Leu Gln Leu Glu Ala Val Asn Ile Thr Asp Leu Ser Glu Asn
        105                 110                 115
   AGA AAG CAG GAC AAG CGC TTC GCC TTC ATC CGC TCA GAC AGT GGC CCC     438
   Arg Lys Gln Asp Lys Arg Phe Ala Phe Ile Arg Ser Asp Ser Gly Pro
   120                 125                 130                 135
   ACC ACC AGT TTT GAG TCT GCC GCC TGC CCC GGT TGG TTC CTC TGC ACA     486
   Thr Thr Ser Phe Glu Ser Ala Ala Cys Pro Gly Trp Phe Leu Cys Thr
                   140                 145                 150
   GCG ATG GAA GCT GAC CAG CCC GTC AGC CTC ACC AAT ATG CCT GAC GAA     534
   Ala Met Glu Ala Asp Gln Pro Val Ser Leu Thr Asn Met Pro Asp Glu
               155                 160                 165
   GGC GTC ATG GTC ACC AAA TTC TAC TTC CAG GAG GAC GAG TAGTAC          579
   Gly Val Met Val Thr Lys Phe Tyr Phe Gln Glu Asp Glu
           170                 175                 180
(2) INFORMATION FOR SEQ ID NO: 14:
   (i) SEQUENCE CHARACTERISTICS:
      (A) LENGTH: 180 amino acids
      (B) TYPE: amino acid
      (D) TOPOLOGY: linear
   (ii) MOLECULE TYPE: protein
   (xi) SEQUENCE DESCRIPTION: SEQ ID NO: 14:
   Met Ala Leu Ala Asp Leu Tyr Glu Glu Gly Gly Gly Gly Gly Gly Glu
    1               5                  10                  15
   Gly Glu Asp Asn Ala Asp Ser Lys Glu Thr Ile Cys Arg Pro Ser Gly
               20                  25                  30
   Arg Lys Ser Ser Lys Met Gln Ala Phe Arg Ile Trp Asp Val Asn Gln
           35                  40                  45
   Lys Thr Phe Tyr Leu Arg Asn Asn Gln Leu Val Ala Gly Tyr Leu Gln
       50                  55                  60
   Gly Pro Asn Val Asn Leu Glu Glu Lys Ile Asp Val Val Pro Ile Glu
   65                  70                  75                  80
   Pro His Ala Leu Phe Leu Gly Ile His Gly Gly Lys Met Cys Leu Ser
                   85                  90                  95
   Cys Val Lys Ser Gly Asp Glu Thr Arg Leu Gln Leu Glu Ala Val Asn
               100                 105                 110
   Ile Thr Asp Leu Ser Glu Asn Arg Lys Gln Asp Lys Arg Phe Ala Phe
           115                     120                     125
   Ile Arg Ser Asp Ser Gly Pro Thr Thr Ser Phe Glu Ser Ala Ala Cys
       130                     135                     140
   Pro Gly Trp Phe Leu Cys Thr Ala Met Glu Ala Asp Gln Pro Val Ser
   145                     150                     155                     160
   Leu Thr Asn Met Pro Asp Glu Gly Val Met Val Thr Lys Phe Tyr Phe
                   165                     170                     175
   Gln Glu Asp Glu
               180

## Claims

1. A substantially pure protein having antagonist activity against at least one of interleukin-1α and interleukin-1β and comprising the amino acid sequence of SEQ ID NO:12.

2. A protein as claimed in claim 1, which comprises the amino acid sequence of SEQ ID NO:14.

3. Isolated DNA encoding a protein having the amino acid sequence of SEQ ID NO: 14.

4. Isolated DNA as claimed in claim 3, which has the sequence of SEQ ID NO: 13.

5. A vector comprising the DNA as claimed in claim 3 or claim 4.

6. A host cell transfected with the vector claimed in claim 5.

7. A host cell as claimed in claim 6 which is of mammalian origin.

8. A process for obtaining an IL-1 antagonist comprising:
a. cultivating a host cell as claimed in claim 6 or claim 7; and
b. collecting and isolating the purified protein.

9. A protein as claimed in claim 2 for use in medicine.

10. The use of a protein as claimed in claim 2 in the preparation of a pharmaceutical compound for use in the prophylactic and/or therapeutic treatment of rheumatoid arthritis, septic shock, acute myelomonocytic leukemia, immunological reaction of transplantation against host, acquired immunodeficiency syndrome (AIDS) or ulcerative colitis.

## Patentansprüche

1. Im wesentlichen reines Protein, welches eine antagonistische Wirkung gegen wenigstens eines von Interleukin-1α und Interleukin-1β aufweist und die Aminosäuresequenz von Seq. ID Nr. 12 umfasst.

2. Protein nach Anspruch 1, welches die Aminosäuresequenz von Seq. ID Nr. 14 umfasst.

3. Isolierte DNA, welche für ein Protein codiert, das die Aminosäuresequenz von Seq. ID Nr. 14 aufweist.

4. Isolierte DNA nach Anspruch 3, welche die Sequenz von Seq. ID Nr. 13 aufweist.

5. Vektor, umfassend die DNA nach Anspruch 3 oder Anspruch 4.

6. Wirtszelle, transfiziert mit dem Vektor nach Anspruch 5.

7. Wirtszelle nach Anspruch 6, die von einem Säuger stammt.

8. Verfahren, um einen IL-1 Antagonisten zu erhalten, umfassend:
a) Kultivieren einer Wirtszelle nach Anspruch 6 oder Anspruch 7; und
b) Sammeln und Isolieren des gereinigten Proteins.

9. Protein nach Anspruch 2, zur Verwendung in der Medizin.

10. Verwendung eines Proteins nach Anspruch 2, zur Herstellung einer pharmazeutischen Verbindung zur Verwendung in der prophylaktischen und/oder therapeutischen Behandlung von rheumatoider Arthritis, septischem Schock, akuter myelomonocytärer Leukämie, immunologischer Transplantatabstoßung, erworbenem Immundefekt-Syndrom (AIDS) oder Colitis ulcerosa.

## Revendications

1. Protéine substantiellement pure ayant une activité antagoniste de au moins l'interleukine-1α ou l'interleukine-1β et comprenant la séquence en acides aminés SEQ ID NO: 12.

2. Protéine telle que revendiquée dans la revendication 1, comprenant la séquence en acides aminés SEQ ID NO: 14.

3. ADN isolé codant une protéine ayant la séquence en acides aminés SEQ ID NO: 14.

4. ADN isolé tel que revendiqué dans la revendication 3, ayant la séquence en acides aminés SEQ ID NO: 13.

5. Vecteur comprenant l'ADN tel que revendiqué dans la revendication 3 ou 4.

6. Cellule hôte transfectée par le vecteur tel que revendiqué dans la revendication 5.

7. Cellule hôte telle que revendiquée dans la revendication 6, qui est d'origine mammalienne.

8. Procédé d'obtention d'un antagoniste de l'IL-1, comprenant:
a. la culture d'une cellule hôte telle que revendiquée dans la revendication 6 ou 7; et
b. la récupération et l'isolement de la protéine purifiée.

9. Protéine telle que revendiquée dans la revendication 2 pour une utilisation en médecine.

10. Utilisation d'une protéine telle que revendiquée dans la revendication 2 pour la préparation d'un composé pharmaceutique pour une utilisation dans le traitement prophylactique et/ou thérapeutique de la polyarthrite rhumatoïde, le choc septique, la leucémie myélomonocytique aiguë, la réaction immunologique de transplants contre l'hôte, le syndrome d'immunodéficience acquise (SIDA) ou la recto-colite hémorragique.
